# EUROPEAN PATENT SPECIFICATION

(11) **EP 0 815 896 B1**
(45) Date of publication and mention of the grant of the patent: **31.10.2001**
(21) Application number: 97110451.8
(22) Date of filing: 26.06.1997
(51) Int. Cl.: A61M 25/10, A61M 25/00, A61N 1/362, A61N 1/365

(54) **System for the treatment of hyperkinetic atrial arrhythmia**
System zur Behandlung einer hyperkinetischen Vorhofarrythmie
Système pour traiter une arrythmie d'oreillette hypercinétique

(30) Priority: 27.06.1996 IT FI960154
(43) Date of publication of application: 07.01.1998
(73) Proprietor: Del Giglio, Mauro, 52022 Cavriglia, (Arezzo) (IT)
(72) Inventor: Del Giglio, Mauro, 52022 Cavriglia, (Arezzo) (IT)
(74) Representative: Bosotti, Luciano

(56) References cited:
- EP-A- 0 335 205
- EP-A- 0 429 141
- US-A- 3 981 299
- US-A- 4 146 029
- US-A- 4 723 946
- US-A- 4 973 319

## Description

### Prior art

Hyperkinetic atrial arrhythmia includes a large number of generally benign disorders characterised by an increase in the frequency of atrial contraction, possibly accompanied by an increase in ventricular frequency leading to fibrillation and flutter in which the movement of the atrium is chaotic and does not correspond to that of the ventricle.

Clinical electrocardiograms may suggest the following risks for the patient:-
1) atrial tachycardia (from 140 to 220 bpm) caused by heart disease: with a net reduction in the cardiac output for reduced atrial and ventricular filling: with hypotension and lipothymia;
2) atrio-ventricular junctional tachycardia with feedback of the P wave and a reduction in cardiac output;
3) multiform supraventricular tachycardia from different atrial pacemakers functioning separately, with extreme variability of cardiac frequency;
4) atrial flutter, in which the frequency of atrial contraction is more than 200 bpm, with a variable ventricular response depending on the conduction ratio (2:1, 3:1 etc);
5) atrial fibrillation, comprising a chaotic movement of the atria which become distended and do not empty properly.

Among these atrial tachyarrhythmias, flutter and fibrillation are without doubt the most disabling.

In particular, atrial fibrillation is an arrhythmia which causes the following main consequences.

From the haemodynamic point of view, there is an absence of atrial systole which, in a normally functioning heart, increases ventricular filling by increasing both the velocity and the volume of blood flow. Its absence therefore reduces cardiac output.

AF leads to a pooling of the blood in the atrium, which favours the formation of thromboembolism which is life-threatening to the patient.

The irregularity of the ventricular response to the chaotic electrical activity in AF may also lead to unrestrained ventricular tachyarrhythmia, which is even more dangerous than atrial tachyarrhythmia.

The treatment of AF (and in the case of type B, of actual flutter also) consists in correcting the sinusal rhythm by cardioversion in the acute or paroxysmal forms; and where cardioversion cannot be used (for example, in chronic forms) the rate is controlled and anticoagulants are administered to reduce the risk of embolism.

Cardioversion may be pharmacological or electrical: for this latter a suitable anticoagulation procedure is necessary with an anaesthetic or sedation being important before administration of the electric shock.

Pharmacological cardioversion also requires anticoagulation, but not anaesthesia, and gives rise to a longer therapeutic effect. Anticoagulation is not necessary if cardioversion is performed in the first 48 hours after the onset of arrhythmia.

### Description

Wherever possible, the cardioversion of atrial fibrillation (AF) by means of intravenous drug infusion has been and is usually applied to patients in which the problem has only recently arisen, or where they have been subjected to treatment with anticoagulant drugs.

From EP-A-0 429 141 an implantable pharmacological defibrillator is known providing for the automatic recognition of ventricular fibrillation. The defibrillator in question includes a catheter insertable through the venous system to the coronary sinus, supporting distally one balloon and provided with a perfusion lumen. The defibrillator also has associated therewith apparatus contained in a casing with an inert seal comprising a reservoir for the drug, a drug infusion system, a circuit for sensing electrical activity connected to the sensors as well as a port for filling the drug reservoirs.

Since it is not always possible to be immediately aware of the onset of AF and, therefore, to intervene with normal procedures, the present invention, having the features set forth in the annexed claims, proposes pharmacological atrial cardioversion to be effected by means of a device which can be implanted like a normal cardiac pacemaker so that at the onset of AF (detected by an electrical, electromechanical or similar sensor) the quantity and the type of drug necessary to obtain cardioversion of the atrium will be injected into the coronary sinus (CS), or at the edge of the right atrium (RA) or the right ventricle (RV).

It is possible to show (and has already been shown experimentally by us) that infusion of the drug through the CS reduces significantly the quantity of drug necessary for defibrillation, thus also reducing the possibility of secondary effects which could arise as the result of heavy drug use, as is necessary with the intravenous approach, and even in atrial or ventricular infusion.

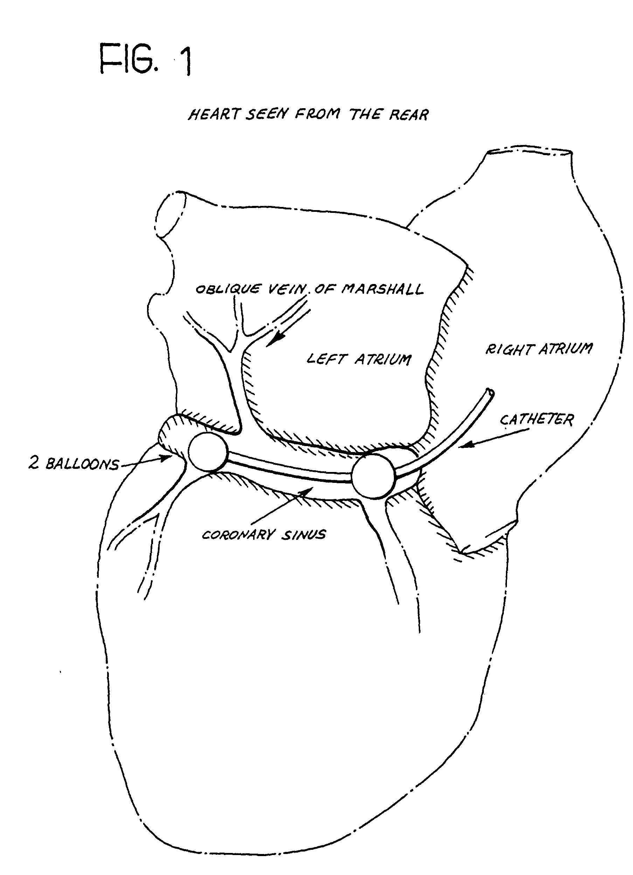

In fact, CS infusion with the system which will be described below enables direct coronary diffusion from the CS, by virtue of the presence of the vein of Marshall which carries blood directly from the atrial wall into the CS itself (Figure 1) and which can therefore transport the infused drug backwards in the CS.

An implantable system is therefore proposed including:
1) - a catheter insertable through the venous system passing, for example, from the superior vena cava to the CS. The catheter must be constructed with a lumen capable of perfusing the drug, and a lumen for inflating and deflating one or more balloons which, during the infusion, create the conditions for the drug to reach the coronary arteries and the vein of Marshall. The possible configurations of the distal part of the catheter to be located in the CS will be described below.
   Where the drug is delivered to the atrium or ventricle, the catheter will only have the lumen necessary for the infusion of the drug, and the outlet hole will open into the atrium or ventricle, protected by a non-return valve or similar systems.
   As well as the lumens necessary for the hydraulic functions, one or more conductors are envisaged which are connected to electrodes and/or sensors which detect the condition of AF and which, alternatively or contemporaneously, are used to stimulate the heart or just the atrium in arrhythmic situations.
2) - a container made from biocompatible material and having an inert seal to which the electrocatheter described above is connected including:- a) a reservoir for the drug; b) a system for inflating and deflating the vessel closure balloon or balloons, c) a system for the infusion of a bolus of drug in a programmable quantity; d) a sensing system for detecting AF and therefore cardiac activity, connected to electrical or electromechanical sensors; e) a system for the electrical stimulation of the heart in arrhythmic emergencies; f) a two-way telemetry system for setting the infusion, sensing and stimulation parameters; g) a valve port for filling the necessary drug reservoir from outside the body.
3) - external telemetric instrumentation for programming the implanted system, which is operatively similar to that used in existing cardiac pacemakers.

The system operates as follows.

The AF sensor, preferably located in the CS or the atrium, detects the onset of AF and, after a short interval, initiates treatment in the following sequence:-
the balloon/balloons are inflated; a suitable pump injects the drug at the programmed pressure and quantity; after a short interval (several seconds) the balloon/balloons are deflated, thereby re-establishing the passage through the CS.

The treatment described above may be effected, according to necessity and the opinion of the cardiologist, with the following alternatives:- a) by means of a single bolus of drugs which perfuses the tissue at the envisaged and calculated quantity; b) with a timed series of reduced infusions so as to achieve the therapeutic quantity, building up the effect of the drug.

This second possibility appears to be better tolerated and less risky since the possible collateral noxious effects of the drug may be more easily avoided or compensated for by prematurely interrupting the infusion.

In this embodiment, the system repeats the sequence of inflating the balloons, perfusing the drug and deflating the balloons several times.

This sequence can obviously be programmed in terms both of the quantity of the drug for each infusion, and the time interval between consecutive operations.

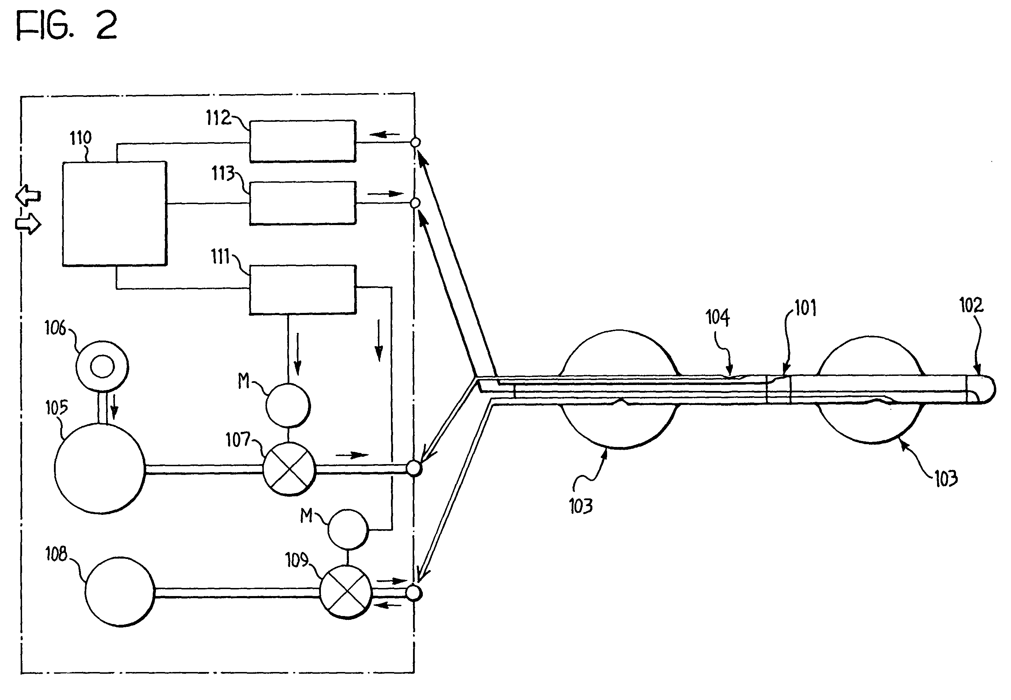

Figure 2 shows schematically a system implantable according to points 1) and 2) discussed above, preferably in the CS.

Only the distal section of the electrocatheter is shown.

This system envisages the catheter being fitted with two balloons 103, controlled simultaneously by means of the fluid pushed through the appropriate lumen, in communication with a hole for each balloon.

One or more sensors 101 for detecting atrial electrical activity and AF are envisaged: the electrode 102 is envisaged for emergency stimulation.

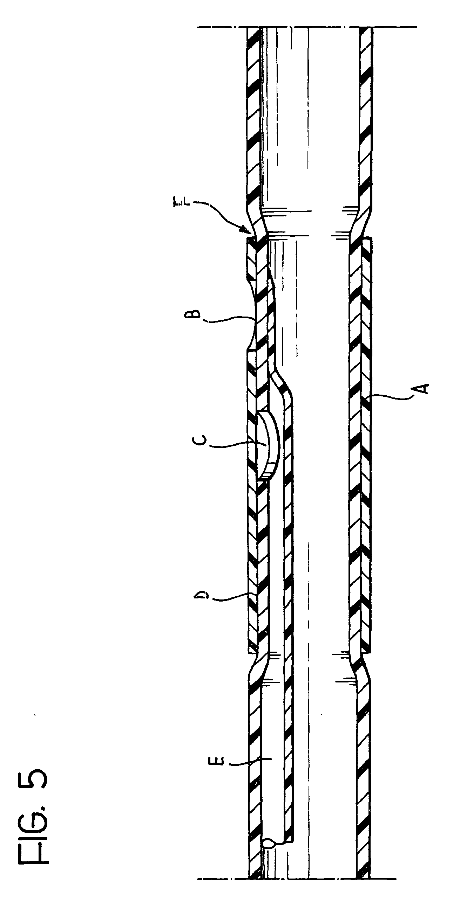

The balloons are positioned on the catheter such that the distal balloon occludes the vena magna where it arises in the CS, and the proximal balloon occludes the entry of the CS into the right atrium. When the balloons are inflated a sort of chamber is formed into which the oblique vein of Marshall opens; the drug injected at a relative pressure is forced into the aforesaid vein (see also Figure 5 and its description).

The operating circuit is contained in a sealed, biocompatible box, connectable to the electrocatheter, which contains: - a supply battery, not shown in the drawing; a drug reservoir 105 which can be filled from outside the body through the permeable gate 106; an electrically controlled pump 107 for the infusion, whose operative characteristics and operation time are programmed depending on the dose of the drug; a small reservoir 108 containing a biocompatible liquid or inert gas for inflating the balloons 103; a reversible pump 109 for inflating and deflating the balloons; a circuit 111 for controlling the hydraulic system; a circuit 112 connected to the sensor 101 for detecting atrial activity and AF; a circuit 113 for emergency stimulation; and a circuit 110 for programming the system with characteristics imposed telemetrically from outside the body.

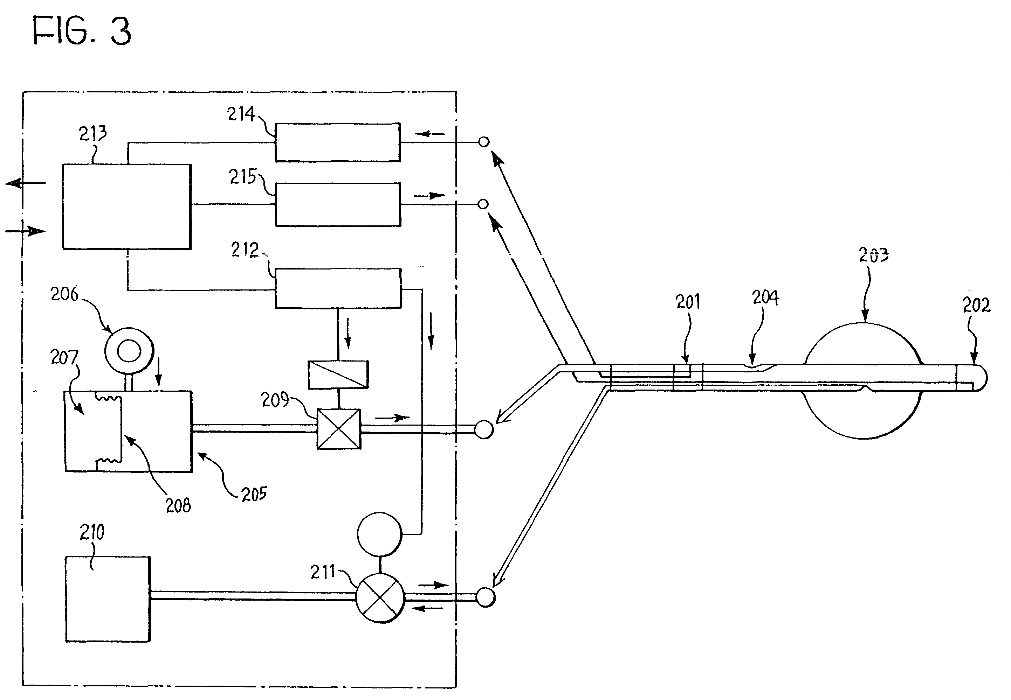

A different embodiment, non-limitative like the other example, of the proposed implantable system is schematically shown in Figure 3, also for CS infusion.

Only the distal part of the electrocatheter is shown, in a possible non-limitative embodiment, which here envisages:- one or more sensors 201 for detecting atrial activity and AF; an electrode 202 for possible stimulation; a balloon 203 disposed distally downstream of the vein of Marshall for closing the input of the vena magna; and the infusion of the drug through an appropriate lumen 204.

The active part of the implantable instrument comprises:- a battery for the electrical and electronic components, not shown in Figure 3; a drug reservoir 205 which can be filled from the outside through the port 206 of known type; as in the other embodiment of the infusion system, in the same container there is a section 207 filled with an inert gas (helium or similar) under pressure separated from the liquid drug by a wall 208 which is a resilient membrane; a solenoid valve 209 controlled by the electronic system, which opens for the time necessary for administration of the drug; a small reservoir 210 containing an inert liquid or gas for inflating the balloon or balloons; an electrically driven pump 211 which alternatively inflates and deflates the balloon when the programme requires it; a circuit 212 for controlling the hydraulic system driven by the programme inserted in the circuit 213; a programming circuit 213 whose characteristics are imposed telemetrically from the outside; a circuit 214 for sensing atrial activity and AF; and a circuit 215 for emergency stimulation.

The various components of the system such as, for example, the infusion system, may be used regardless of their other possible uses.

The construction of the electrocatheter, as shown in Figures 2 and 3, depends on the arrangement chosen for the infusion of the drug into the CS.

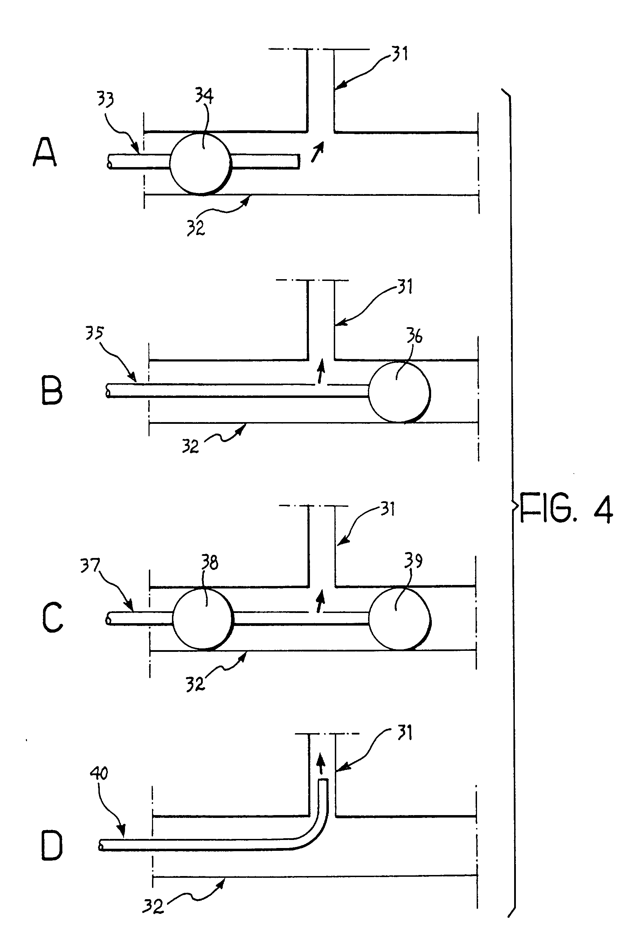

Figure 4 represents schematically several possible embodiments of this solution.

In Figure 4, the CS is indicated 32, and the oblique vein of Marshall which arises from the left atrium is indicated 31. The drawings show various systems for obtaining the infusion of the drug in the atrial wall.

In the systems A, B and C, the arrangement of the hydraulic ducts does not vary with respect to that shown in Figures 2 and 3, apart from the fact that the lumen which carries the fluid for inflating the balloons has to reach every single balloon, and the lumen for the infusion of the drug must be open in the predetermined zone.

On the other hand, the system D envisages a catheter dedicated to infusion which is orientable for the cannulation of the vein of Marshall or just the CS.

Analysing the various systems:-
A - In this case, the balloon 34 occludes the entry of the CS in the right atrium; by injecting the drug and also considering the venous pressure arising from the coronary veins (vena magna), the drug will be forced into the vein 31, but also partly into the ventricles.
B - The balloon 36 closes the CS downstream of the vein 31 (with respect to normal blood flow). Perfusion through the catheter 35 at the correct pressure encourages the entry of the drug into the vein 31, although with some loss to the right atrium.
C - This system envisages two balloons in communication with the same lumen which inflates them at the same time; 38 and 39 thus create a chamber which facilitates the infusion of the drug in the vein 31.
D - As indicated, this system would require a catheter 40 sufficiently flexible and thin as to be able to cannulate the vein 31 or just the CS. In this case it might be necessary to use a different catheter to support the sensor and the stimulation electrode.

The following problem must be resolved.

For long-term implantation, the catheter must be constructed in such a way to avoid the formation of clots on its structure, especially where the outlet hole corresponding to the lumen which carries the drug is formed.

To avoid blood passing through the hole into the lumen, with possible coagulation and therefore occlusion, the use of a valve is proposed which, as a non-limitative example, may be formed as shown schematically in Figure 5.

On a section of the catheter which includes the outlet hole C, appropriately shaped for uniformity of diameter, is a segment of tube A of a resilient biocompatible material, with a very thin wall and a hole at one end (B). The position of this sleeve is such that the outlet hole C is covered and the hole B does not coincide with the hole C on the catheter. The sleeve is fitted to the end D opposite the hole B which must obviously be free.

When the pump delivers the drug through the appropriate lumen E, the pressure exerted will be set such that it can lift the free edge of the resilient sleeve, permitting the liquid to exit from the hole B and/or the free end (F) of the sleeve itself.

In order always to prevent blood entering the catheter and/or coagulating at the drug outlet, various means may be used, specifically by avoiding any valve structure and acting on the structure of the catheter itself.

A "porous" catheter segment can be inserted in the section of the catheter from which the drug must be released: the structure of the catheter material may be made permeable using various techniques, creating a porosity with holes compatible in size with the molecular structure of the drug to be infused (for example, with diameters of approximately 20 *µ*m). The drug is forced out by means of a suitable pressure.

The advantages in using a system like the proposed system lie in the timeliness of the therapeutic intervention which protects the patient from pooling of blood in the atrium, thus removing the risk of the formation and propagation of thromboses: and in the possibility of pharmacological intervention at the onset of AF, when the intervention threshold is lower, thus enabling the use of smaller quantities of the drug in comparison with later intervention, as currently occurs with the pharmacological or electrical treatments currently practised.

Particularly where a catheter for the atrial injection of the drug is envisaged, the best scheme is that of Figures 2 and 3 in which, however, the operation of the block 110 or 213, and the adjacent blocks must be implemented, and in which the catheter no longer carries the balloons. The mechanical simplification of the catheter is balanced by the greater complexity of the necessary program for the CPU which must be contained in 110 or 213.

In fact, the introduction of the drug into the atrium requires a greater quantity of drug, approximately 5-6 times that necessary in the CS.

This increases the likelihood of collateral effects of the drug, which makes the strict monitoring of the electrical (and mechanical) characteristics of the heart necessary. However, such monitoring is also useful and advisable even for the CS solution

The electrical characteristics to be monitored are:- a) cardiac rate; b) QRs duration; c) the distance PQ; d) QTc length; e) the onset of A-V block; f) cardiac muscle contractility.

In the embodiment of the proposed system, all or just some of the aforesaid parameters may be monitored.

These parameters may be detected by means of electrodes and sensors positioned on the catheter so as to detect the atrial electrical activity, ventricular electrical activity, and muscular activity.

If the electrocatheter (also including the drug-infusion lumen) with an electrode at the distal end is positioned in the RV, and with electrodes nearby in the RA, the logistic situation is similar to that of the "single lead" electrocatheters for VDD stimulation. In this case, the lumen for the drug opens into the RA for atrial infusion, or it could open into the RV for entry of the drug into the circulation. The contractility sensor is disposed distally in the ventricle. This could be the BEST sensor designed by the firm SORIN BIOMEDICA SpA.

On the other hand, where the electrocatheter is inserted in the CS with the associated balloons, atrial activity may be detected by electrodes situated in the CS, while ventricular activity may be detected by extending the catheter to the vena magna, with a distally disposed electrode, together with the contractility sensor.

With these possible arrangements, all of the electrical signals necessary to obtain the parameters discussed above are available: they may be sent to the CPU for processing so as evaluate the necessary parameters.

The programme for telemetrically connecting with or sending to the CPU must provide for a break in supply if the parameters vary outside the programmed thresholds:-
a) an increase or decrease in cardiac rate from the predetermined thresholds and/or possible arrhythmia: the infusion of the drug is stopped and emergency stimulation may be activated;
b) an increase in QRS duration: infusion stops;
c) an increase in PQ: infusion stops;
d) an increase in QTc: infusion stops;
e) onset of AV block: infusion stops;
f) a reduction in contractility; infusion stops.

An instrument such as that described may advantageously be formed with a system which uses a microcontroller (or a microprocessor) in order to obtain maximum functional flexibility.

This arrangement is practically obligatory when it is necessary to analyse different parameters, and to provide feedback of the infusion characteristics and possible stimulation.

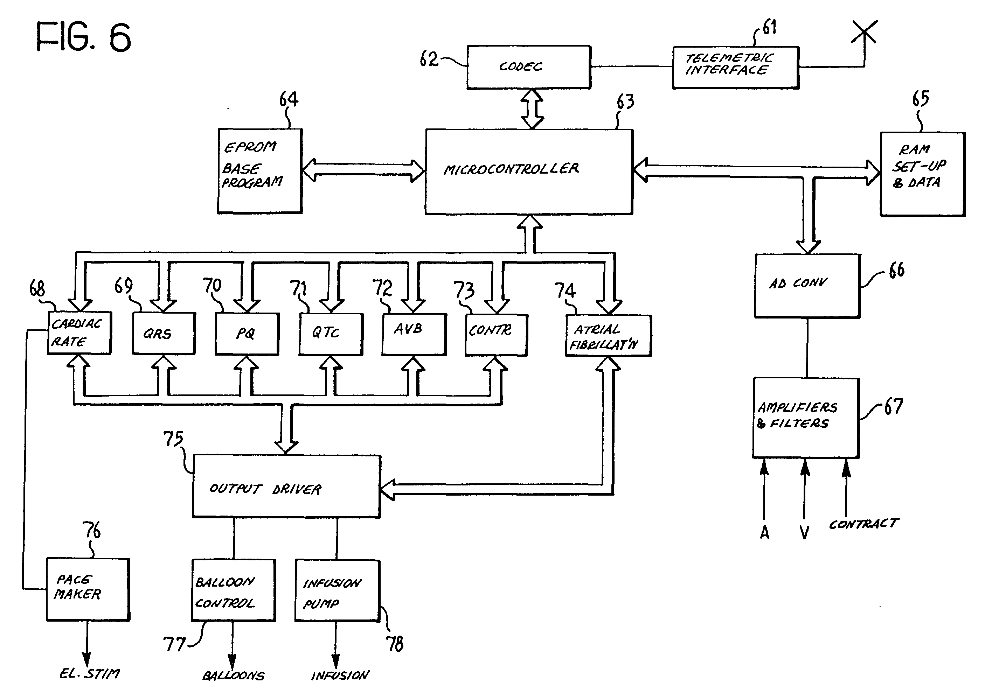

Figure 6 shows a possible structure for the proposed system.

A telemetric interface 61 enables the acquisition of data or the sending of commands by means of a coupling (electric, electromagnetic, RF) to and from the outside, via a coder/decoder (CODEC) 62 which joins with the transmitter-receiver outside the body.

The base programme contained in the EPROM memory 64 uses the commands received by telemetric means to control, by means of the microcontroller 63, the drug infusion and emergency stimulation programmes.

These programmes are controlled by the interfaces 68-74 which relate to the parameters of the signals detected through the electrocatheter, and the amplifiers and filters contained in the block 67, converted by the A/D block 66.

The interface 74, in the presence of ECG signals with AF, activates the outlet driver 75 for the drug infusion cycle. In this circumstance, the block 77 provides for the inflation and deflation of the balloons (when they are present) and the block 78 specifies the drug infusion cycle.

The interfaces 68-73 which contain the intervention thresholds will interrupt the infusion cycle when these thresholds are exceeded.

In the case of intervention of the interface 68, which relates to cardiac rate, in the case of cardiac arrest or bradycardia below the threshold, it will enable emergency stimulation by means of the block 76, with the characteristics inserted in the programme 64.

All of the data relating to the parameters received telemetrically and which constitute the SET UP of the infusion-stimulation system are stored in the RAM 65 which also stores the data acquired from the A/D interface 66 and processed by the microcontroller 63 for the various interfaces 68-74.

It is thus possible to retransmit to the outside, coded by 62, both the state of the parameters of operation and the received and processed data.

It is noted that there are currently available integrated systems which contain the majority of the blocks indicated in the drawings, as well as the microcontroller, in a single chip, with advantages of storage and energy supply.

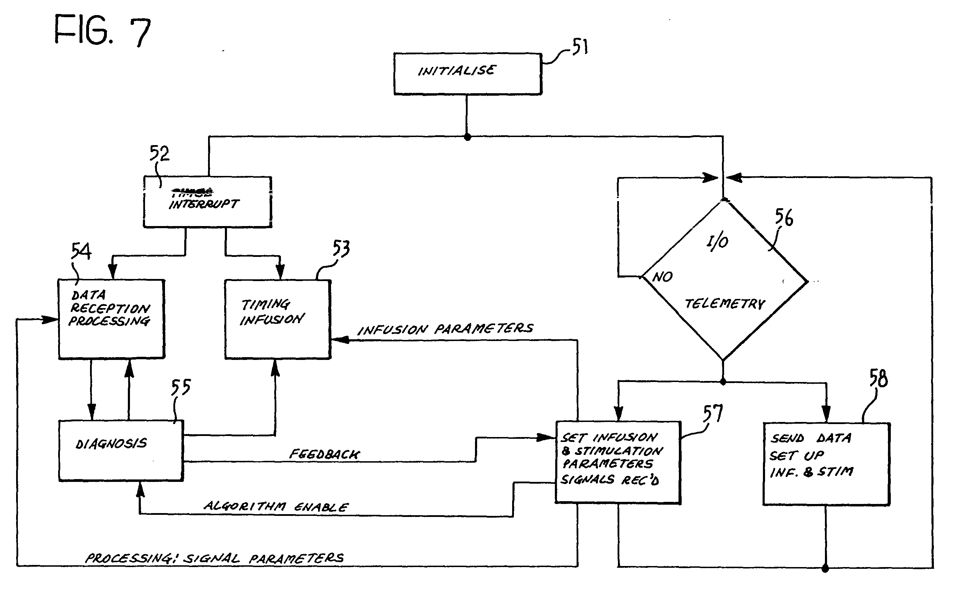

A possible flow chart of the software for the system of Figure 6 is shown in Figure 7.

The initialisation block 51 comes into play at the beginning of the system. It activates all the peripherals (timer, data transmission and reception etc), and imposes the default parameters for drug infusion, for processing the required cardiac parameters, and for possible feedback between the received signals and infusion. This data can then be modified telemetrically by the user.

The real-time proceses to be realised are represented by the blocks 53 and 54. A timed interrupt 52 activates the processes of these blocks with absolute priority. The timer must be set correctLy for the frequency at which the systems for the A/D conversion of the block 54 must operate, being those for the infusion and the generation of emergency stimulation impulses by the block 53.

The timer generates an interrupt for the CPU in such a way as to give precedence to these processes.

The block 54 is able to accumulate the data necessary for the processing of the parameters in the area of storage and subsequently returns them to the block 55 which contains the appropriate diagnostic algorithms and the consequent feedback on the infusion and emergency stimulation parameters. These functions may be performed or modified by the external user.

In the interval between the interrupt, the CPU tests the telemetric I/O interface 56 to establish whether there is a requirement for data (which will be provided by the block 58) or a command code for the imposition or modification of the infusion or stimulation parameters by the block 57.

In the case of the required data (cardiac parameters, ECG, the state of the infusion or stimulation data), the block 58 is able to send data to the coding and telemetry system.

In the case of imposition or modification of the intervention parameters, the block 57 will from time to time set infusion or stimulation data, or data for acquisition of external signals (parameter type, signal type etc).

When the interaction between the signals received and the decision to intervene is effected, the diagnostic block 55 is able to act with the required algorithim, activating feedback with the block 57.

In addition to being used for cardioverting the aforesaid atrial arrhythmia, the system may be used for the administration of cardioactive drugs which act specifically on the myocardium or on its conduction apparatus. In these cases, it is important that the administration programme, in the case of a system used as that described, is easily adaptable to the needs of the patient by telemetric means, while also retaining the monitoring of the electromechanical characteristics of the heart, which react to the infusion.

## Claims

1. An implantable system for performing cardioversion of atrial fibrillation (AF) and/or atrial flutter or other disabling arrhythmias, through the infusion of an anti-arrhythmical or anti-fibrillation drug into the coronary sinus or in the right atrium or right ventricle, including:
- a catheter insertable through the venous system to the coronary sinus, supporting distally one or two balloons (103, 203) adapted to occlude the vena magna or the entry of the coronary sinus (CS) into the right atrium, including a lumen for inflating the said balloons whereby, when the balloon/balloons are inflated, a sort of chamber is formed into which the oblique vein of Marshall opens, and a lumen (104, 204) opening out into said chamber for the perfusion of the drug towards the vein of Marshall when the catheter is inserted,
- one or more conductors connected to electrodes and/or sensors (101, 201) for detecting AF and which are able to stimulate the heart;
- apparatus contained in a casing with an inert seal comprising: a reservoir (105, 205) for the drug; a system (108, 109; 208, 209) for inflating and deflating the balloon/balloons; a drug infusion system (107; 207); a circuit (112; 212) for sensing atrial electrical activity connected to the catheter sensors; an electrical stimulator (102, 113; 202, 215) for emergency arrhythmia; a port (106; 206) for filling the drug reservoir, whereby said system is configured for performing cardioversion immediately following the onset and detection of arrhythmia, and also provides for the monitoring of the electromechanical cardiac parameters for controlling the infusion of the drug.

2. A system as in Claim 1, in which the drug infusion system comprises a sealed chamber divided internally into two parts (205, 207) by a resilient wall (208); an inert gas under pressure being contained in one (207) of these parts; and the drug to be urged towards the catheter by the gas pressure when a communication valve (209) opens being contained in the other (205) of these parts.

3. A system as in Claim 1, in which the drug infusion system is constituted by a reservoir (105) connected to a pump (107) which urges the drug contained in the reservoir towards the infusion lumen of the catheter.

4. A system as in Claim 1, in which the balloon/balloons (103; 203) are inflated and deflated by a reversible pump (109; 211) which urges the fluid, gas or liquid, through the catheter.

5. A system as in Claim 2, in which a programmable control circuit is provided for the dosage of the drug, said control circuit timing the opening of the valve (209) between the drug reservoir (105; 205) and the catheter.

6. A system as in Claim 3, in which a programming circuit (213) is provided for the dosage of the drug, said circuit (213) programming telemetrically (61) the speed or the firing time (75) of the pump (107).

7. A system as in Claim 1, in which the catheter includes an outlet hole (C) and a valve (A, B, F) associated with the outlet hole (C), said valve being comprised of a segment of a tube of a resilient biocompatible material covering said outlet hole (C) and provided with a respective hole (B) offset with respect to said outlet hole (C) on the catheter.

8. A system as in Claim 1, in which the catheter is constituted in correspondence with the drug ejection zone by a segment having a porous structure, with holes for the release of the drug which are determined by its composition so as to be able to force the drug out at a predetermined pressure, but preventing blood from entering the catheter.

9. A system as in Claim 1, comprising a cardiac monitoring system for the protection of the patient for monitoring, during infusion, specific parameters for each type of drug that can be used such as, for example, cardiac rate, QRS duration, PQ segment, AVB situation, deterioration in cardiac contractility; infusion being stopped if the parameters change beyond the predetermined thresholds.

10. A system as in Claim 1, in which the system is configured for the administration of small drug boluses in a timed sequence, such as one per minute, in such a way that the sum of the boluses corresponds to the dose necessary for cardioversion.

11. A system as in Claim 1 **characterised in that** the system includes means (74) for detecting the onset of AF and is configured for bringing about the action of cardioversion some seconds after the onset of AF, enabling the object to be achieved with the minimum quantity of drugs, thereby avoiding the risk of blood stagnating in the atrium, and diminishing the collateral effects of the drug.

## Patentansprüche

1. Ein implantierbares System zum Durchführen einer Cardioversion bei Vorhofflimmern (AF) und/oder Vorhofflattern oder anderen beeinträchtigenden Arrhythmien anhand der Infusion von antiarrhythmischen oder antifibrillatorischen Medikamenten in den Coronarsinus oder in den rechten Vorhof oder in die rechte Kammer, wobei das implantierbare System umfaßt:
einen Katheter, der durch das venöse System in den Coronarsinus eingeführt werden kann und der distal einen oder zwei Ballons (103, 203), die zum Verschließen der Vena magna oder der Mündung des Coronarsinus (CS) in die rechte Kammer geeignet sind, trägt, der zudem ein Lumen zum Aufblasen dieser Ballons aufweist, wobei bei aufgeblasenem Zustand des/der Ballons eine Art Kammer gebildet wird, in welche sich die Marshall-Vene öffnet, und ein Lumen (104, 204), welches sich in diese Kammer zur Perfusion des Medikaments in die Marshall-Vene eröffnet, wenn der Katheter eingeführt wird,
einen oder mehrere Leiter, die mit Elektroden und/oder Sensoren ((101, 201) zur Erfassung von AF verbunden sind und das Herz stimulieren können;
eine in einem Gehäuse angeordnete Vorrichtung mit einer Schutzdichtung, die aufweist: einen Vorratsbehälter (105, 205) für das Medikament; ein System (108, 109; 208, 209) zum Aufblasen und Entleeren des/der Ballons; ein Medikamenteninfusionssystem (107; 207); einen mit den Kathetersensoren verbundenen Kreislauf (112; 212) zum Messen elektrischer Vorhofaktivität; einen elektrischen Stimulator (102, 113; 202, 215) für Notfall-Arrhythmien; eine Schleuse (106; 206) zum Auffüllen des Medikamentenvorratsbehälters, wobei dieses System zur Durchführung einer Cardioversion direkt nach dem Beginn und einer Arrhythmie-Erfassung ausgebildet ist und auch für die Überwachung der elektromechanischen cardialen Parameter zur Steuerung der Medikamenteninfusion sorgt.

2. Ein System nach Anspruch 1, in dem das Medikamenteninfusionssystem eine abgedichtete Kammer, die innen durch eine elastische Wand (208) in zwei Bereiche (205, 207) unterteilt ist, aufweist; wobei sich in einem (207) dieser Bereiche ein unter Druck stehendes Inertgas und im anderen (205) dieser Bereiche das Medikament, welches in den Katheter getrieben wird, wenn ein Verbindungsventil (209) öffnet, befindet.

3. Ein System nach Anspruch 1, in dem das Medikamenteninfusionssystem aus einem Vorratsbehälter (105) besteht, der mit einer Pumpe (107) verbunden ist, die das in dem Vorratsbehälter enthaltene Medikament in das Infusionslumen des Katheters treibt.

4. Ein System nach Anspruch 1, bei dem der/die Ballon/Ballons (103; 203) durch eine umkehrbare Pumpe (109; 211), die das Fluid, das Gas oder die Flüssigkeit in den Katheter treibt, aufgeblasen und entleert wird.

5. Ein System nach Anspruch 2, in dem ein programmierbarer Steuerungskreislauf für die Dosierung des Medikaments, wobei der programmierbare Steuerkreislauf das Öffnen des Ventils (209) zwischen dem Medikamentenvorratsbehälter (105; 205) und dem Katheter zeitlich abstimmt, angeordnet ist.

6. Ein System nach Anspruch 3, in dem ein programmierbarer Kreislauf (213) zum Dosieren des Medikamentes angeordnet ist, wobei der Kreislauf (213) telemetrisch (61) die Geschwindigkeit oder die Auslösezeit (75) der Pumpe (107) programmiert.

7. Ein System nach Anspruch 1, in dem der Katheter eine Auslaßöffnung (C) und ein Ventil (A, B, F), das mit der Auslaßöffnung (C) verbunden ist, umfaßt, wobei das Ventil aus einem Teil eines Rohres aus einem elastischen, biokompatiblen Material besteht, das die Auslaßöffnung (C) überdeckt und eine entsprechende Öffnung (B) aufweist, die am Katheter zur Auslaßöffnung (C) versetzt angeordnet ist.

8. Ein System nach Anspruch 1, in dem der Katheter mit dem Medikamenteninjektionsbereich durch eine poröse Struktur mit Öffnungen zum Auslassen des Medikamentes, welche durch die Zusammensetzung des Medikamentes bestimmt werden, um das Medikament mit einem vorbestimmten Druck heraustreiben zu können, wobei das Blut jedoch daran gehindert wird, in den Katheter einzudringen, in Verbindung steht.

9. Ein System nach Anspruch 1, das ein cardiales Überwachungssystem zum Schutz des Patienten während der Infusion zum Aufzeichnen spezifischer Parameter, wie beispielsweise Herzfrequenz, QRS-Dauer, PQ-Segment, AVB-Situation und Verschlechterung der cardialen Kontraktibilität für jede Medikamentenart, die verwendet werden kann, aufweist; dabei wird die Infusion abgebrochen, wenn die Parameter sich über eine vorher festgelegte Schwelle hinweg verändern.

10. Ein System nach Anspruch 1, wobei das System für die Verabreichung kleiner Medikametenbolusse in einer bestimmten Zeitsequenz, beispielsweise ein Bolus pro Minute, konfiguriert ist, so daß die Summe der Bolusse der für die Cardioversion erforderlichen Dosis entspricht.

11. Ein System nach Anspruch 1, **dadurch gekennzeichnet, daß** es Vorrichtungen (74) zur Erfassung des Beginns von AF aufweist, und derart konfiguriert ist, daß es eine Cardioversion wenige Sekunden nach Eintreten der AF einleitet, wodurch das Ziel mit einer Minimalmenge von Medikamenten erzielt werden kann, wodurch das Risiko der Blutstase im Vorhof vermieden werden kann und die Nebenwirkungen des Medikamentes vermindert werden.

## Revendications

1. Système pour implantation destiné à rétablir le rythme sinusal de la fibrillation auriculaire (AF) et/ou du flutter auriculaire ou d'autres arythmies invalidantes par l'injection d'un anti-arythmique ou d'un anti-fibrillant dans le sinus coronaire ou dans l'oreillette droite ou dans le ventricule droit, incluant :
◆ un cathéter pouvant être inséré à travers le système vasculaire veineux dans le sinus coronaire, supportant de manière distale un ou deux ballonnets (103, 203) ajustés en position d'occlusion de la *venae magna* ou de l'entrée du sinus coronaire (CS) dans l'oreillette droite, incluant une lumière pour le gonflage desdits ballonnets, avec pour effets que, lorsque le ou les ballonnets est ou sont gonflé(s), une sorte de chambre est formée, dans laquelle la veine oblique de l'oreillette gauche s'ouvre, et qu'une lumière (104, 204) s'élargit dans ladite chambre pour l'injection du médicament vers la veine de l'oreillette gauche lorsque le cathéter est inséré ;
◆ un ou plusieurs conducteur(s) relié(s) à des électrodes et/ou à des capteurs (101,201) pour la détection de l'AF et qui sont aptes à stimuler le coeur ;
◆ un appareil logé dans un boîtier muni d'un obturateur inerte comprenant :
• un réservoir (105, 205) destiné au médicament ; un système (108, 109 ; 208, 209) destiné à gonfler ou dégonfler le ou les ballonnet(s) ; un système d'injection de médicament (107, 207) ; un circuit (112, 212) pour détecter l'activité électrique auriculaire relié aux capteurs du cathéter ; un stimulateur électrique (102, 113 ; 202, 215) pour arythmie d'urgence ; un orifice (106 ; 206) pour le remplissage du réservoir, ayant pour effet que ledit système est configuré pour effectuer un rétablissement du rythme sinusal immédiatement après le début et la détection de l'arythmie et assure, en outre, la surveillance des paramètres cardiaques électromécaniques pour réguler l'injection du médicament.

2. Système selon la revendication 1, dans lequel le système d'injection de médicament comprend une chambre rendue hermétique divisée de manière interne en deux parties (205, 207) par une paroi résiliente (208) ; un gaz inerte sous pression étant confiné dans l'une (207) de ces parties ; et le médicament, destiné à être poussé vers le cathéter par la pression gazeuse lorsqu'une valve de communication (209) s'ouvre, étant contenue dans l'autre (205) de ces parties.

3. Système selon la revendication 1, dans lequel le système d'injection de médicament est constitué d'un réservoir (105) relié à une pompe (107) qui pousse le médicament contenu dans le réservoir vers la lumière d'injection du cathéter.

4. Système selon la revendication 1, dans lequel le ou les ballonnet(s) (103; 203) est ou sont gonflé(s) et dégonflé(s) par une pompe réversible (109 ; 211) qui pousse le fluide, le gaz ou le liquide à travers le cathéter.

5. Système selon la revendication 2, dans lequel un circuit de commande programmable est prévu pour le dosage du médicament, ledit circuit de commande cadence l'ouverture de la valve (209) entre le réservoir de médicament (105 ; 205) et le cathéter.

6. Système selon la revendication 3, dans lequel un circuit de programmation (213) est prévu pour le dosage du médicament, ledit circuit (213) programmant par télémétrie (61) la vitesse ou la durée d'activation (75) de la pompe (107).

7. Système selon la revendication 1, dans lequel le cathéter inclut un orifice de sortie (C) et une valve (A, B, F) associée à l'orifice de sortie (C), ladite valve étant constituée d'un segment de tube de matériau biocompatible résilient recouvrant ledit orifice de sortie (C) et muni d'un orifice (B) respectif décalé par rapport audit orifice de sortie (C) ménagée dans le cathéter.

8. Système selon la revendication 1, dans lequel le cathéter est constitué, en correspondance avec la zone d'éjection de médicament, d'un segment possédant une structure poreuse, munie d'orifices pour délivrer le médicament qui sont déterminés par sa composition de sorte à être aptes à pousser le médicament vers l'extérieur à une pression prédéterminée, mais en empêchant le sang de pénétrer dans le cathéter.

9. Système selon la revendication 1 comprenant un système de surveillance cardiaque pour la protection du patient destiné à surveiller, pendant l'injection, les paramètres spécifiques à chaque type de médicament qui peut être utilisé comme, par exemple, le débit cardiaque, la durée du complexe QRS, le segment PQ, la situation AVB, la détérioration de la contractilité cardiaque ; l'injection étant arrêtée si les paramètres changent au-delà des seuils prédéterminés.

10. Système selon la revendication 1, dans lequel le système est configuré pour l'administration d'embols de faible volume au cours d'une séquence temporelle, comme d'un par minute, de manière telle que la somme des embols de médicament corresponde à la dose nécessaire au rétablissement du rythme sinusal.

11. Système selon la revendication 1, **caractérisé en ce que** le système inclut un moyen (74) pour détecter le début d'une AF et est configuré pour déclencher l'action de rétablissement du rythme sinusal quelques secondes après le début de l'AF, en permettant que ce but soit atteint avec le quantité minimale de médicament, avec pour effets d'éviter le danger d'un stagnation sanguine dans l'oreillette et de diminuer les effets secondaires du médicament.
